# EUROPEAN PATENT SPECIFICATION

(11) **EP 2 663 243 B1**
(45) Date of publication and mention of the grant of the patent: **21.10.2015**
(21) Application number: 11701880.4
(22) Date of filing: 12.01.2011
(51) Int. Cl.: A61B 17/064, A61B 17/11, A61F 2/06

(54) **Side to side anastomosis**
Latero-laterale Anastomose
Anastomose latéro-latérale

(43) Date of publication of application: 20.11.2013
(73) Proprietor: Corvasco Medical B.V., 3584 CM Utrecht (NL)
(72) Inventor: TULLEKEN, Cornelis, Antonius, Franciscus, NL-3511 XP Utrecht (NL); TULLEKEN, Rutger, Joan, Bart, NL-1401 AD Bussum (NL)
(74) Representative: EP&C
(86) International application number: PCT/NL2011/050019
(87) International publication number: WO 2012/096563

(56) References cited:
- WO-A1-2004/096059
- WO-A1-2008/046572
- WO-A1-2009/123434
- US-A- 5 964 750
- US-A1- 2002 161 383

## Description

The invention relates to the field of side-to-side (abbreviated as STS) anastomosing.

A STS-anastomosis is a in the medical field a connection between two channels, in general blood vessels, which are connected with their sides against each other. In the region where the sides of the vessels lie against each other there is an aperture allowing blood to flow from the one vessel into the other vessel. STS-anastomoses are frequently used in the field of bypass surgery for example on the heart.

According to the older medical prior art these kind of anastomoses with a graft vessel and recipient vessel are made as follows: in each vessel an opening is made in the vessel wall; these openings are laid onto each other with the openings overlapping each other; and subsequently the graft and recipient vessels are attached to each other by suturing. This is a time consuming operation and in order to prevent spillage of blood the recipient vessel need to be shut off from blood flow - like by clipping -. According to more recent medical prior art connector devices have been developed to simplify making the STS-anastomosis. Up to now, these connector devices do not seem to be used widely spread. Apparently, these connector devices still do not fulfil the needs and requirements of safety and reliability.

The object of the invention is to provide a reliable manner for making an anastomosis. According to a first aspect, this object is achieved by providing an assembly of a graft vessel and an anastomosis connector according to claim 1. According to a second aspect, this object is achieved by providing a method of making (such) an assembly according to claim 12.

All these aspects of the invention have in common, that use is made of an "anastomosis connector of the type comprising a ring and two pins"; wherein each pin has a free end and a fixed end connected to the ring, wherein the pins extend, viewed from the fixed end towards the free end, next to and set apart from each other; and wherein each pin has an overlap section where the pin, viewed in axial direction of the ring, overlaps the plane bounded by the outer contour of the ring.

An "anastomosis connector of the type comprising a ring and two pins" is as such known from WO 2009/123434 the two part form of independent claim 1 is based on this document. Also document WO 2011/062495 describes an anastomosis connector of this type. With respect to these PCT publications, it is noted that the applicant AMJ B.V. of this application is also the applicant of WO 2009/123434 and WO 2011/062495.

Like in WO 2009/123434 and/or WO 2011/062495, also in the "anastomosis connector of the type comprising a ring and two pins" as used in the present invention:
- at least part of said overlap sections can extend parallel to the ring, in a first position of the pins with respect to the ring; and/or
- the pins can be rigidly connected to the ring; and/or
- the pins can extend in the same direction;
- the pins, can with respect to the ring, be moveable from a first position to a second position in order to form between on the one hand the ring and on the other hand the pins an opened jaw; and in which the pins are moveable from the second position to the first position towards the ring in order to close the jaw; and/or
- in the second position, the overlap sections of the pins can be at an angle with respect to the plane defined by the ring in order to form a sad jaw which, viewed from the fixed end towards the free end, widens, and wherein the pins are swivelable, with respect to the ring, from the second position to the first position; and/or
- the anastomosis connector can further comprises a tensioning member which, when the pins are in the second position, pretensions the pins towards the first position; and/or
- the free ends of the pins can be are arranged at a distance from the overlap section, which distance is at least 50% of the radius of the ring, in particular at least 100% of the radius of the ring; and/or
- the sections of the pins between the overlap section and the free end of each pin, can extend essentially parallel to each other.

An assembly according to claim 1 enables making a side-to-side anastomosis connection in an easy manner which requires relative little time and results in a very safe and reliable anastomosis.

With respect to the graft vessel it is to be noted that according to the invention this can be an artificial vessel or a biological vessel. According to the invention, the graft vessel can also be made from a combination of biological and artificial material. In case of a biological vessel, it is noted that this can originate from a human or animal donor - other than the patient itself -, but it can also originate from the patient itself, like a saphenous vein from the leg or an internal mammary artery from the chest. The graft vessel can according to the invention also be a vessel having a biologically cultured cell layer grown onto a supporting tube, like a harness or gauze structure.

Further it is noted that the assembly according to the invention, also in case a graft vessel originating from the patient itself, is prepared completely outside the patient and without being connected in any manner to the patient. The assembly according to the invention can of course be made in the operation room during the operation just beside he patient itself, but it can also be made remote from the patient in a laboratory, factory or other suitable facility during or before the operation.

According to a further embodiment of the assembly according to the invention, the wall of the graft vessel is provided with an opening surrounded by the ring. According to the invention this opening can have been formed by laser cutting an opening into the wall of the graft vessel.

According to a further embodiment of the assembly according to the invention, the connector is attached to the graft vessel by sutures attaching the ring onto the wall of the graft vessel.

According to a further embodiment of the assembly according to the invention, the connector is attached to the graft vessel by adhesive adhering the ring onto the wall of the graft vessel. This attachment by adhesive can according to the invention also be combined with the above mentioned attachment by sutures but the attachment with adhesive can also be without attachment by sutures.

According to a further embodiment of the assembly according to the invention, the connector is attached to the graft vessel by the pins piercing through the wall of the graft vessel. This attachment by the pins can according to the invention also be combined with the above mentioned attachment by sutures and/or adhesive, but the attachment with the pins can also be without attachment by sutures and/or adhesive. Using the pins for attachment of the connector to the graft vessel may allow, after attachment of the assembly according to the invention to the recipient vessel, wall tissue of both the recipient vessel and the graft vessel being clamped between the pins and the ring of the connector. This may improve the tightness of the connection as well the connection being leak free. Further this may support - after the operation - growth of the tissue of the graft vessel and recipient vessel into integral new tissue.

According to a further embodiment of the assembly according to the invention, the pins extend in the longitudinal direction of the graft vessel. This allows the pins to be longer than the diameter of the ring. Pins longer than the diameter of he ring may be practical when attaching the assembly according to the invention to the recipient vessel.

According to a further embodiment of the assembly according to the invention, the ring lies against the outer surface of the wall of the graft vessel. This is easy to establish. Further it allows the operator when attaching the assembly to the recipient vessel a good view on the location of attachment.

According to a further embodiment of the assembly according to the invention, the a part of the wall of the graft vessel lying inside the region surrounded by the ring covers the inside of the ring. The pins might stay uncovered. It might even be folded over 180° around the ring to fully cover the ring whilst leaving the pins uncovered. In this way, internal wall tissue of the graft vessel will prevent blood, flowing through the anastomotic connection, from contacting the ring.

According to a further embodiment of the assembly according to the invention, the ring lies against the inner surface of the wall of the graft vessel and the pins lie outside the graft vessel and extend parallel to the outer surface of the wall of the graft vessel. As indicated above, this may allow, after attachment of the assembly according to the invention to the recipient vessel, wall tissue of both the recipient vessel and the graft vessel being clamped between the pins and the ring of the connector. This may improve the tightness of the connection as well the connection being leak free. Further this may support - after the operation - growth of the tissue of the graft vessel and recipient vessel into integral new tissue.

According to a further embodiment of the assembly according to the invention, the graft vessel has a first end and a second end; wherein the pins, viewed from the fixed ends towards the free ends of the pins, extend towards the second end; and wherein the distance from the connector to the first end of the graft vessel is smaller than the distance from the connector to the second end. This allows when, an applicator is inserted through the same end of the graft vessel as through which the connector has been inserted, good manipulation of the assembly according to the invention as one has a good view onto the pins. Insertion of the applicator and the connector through the same end of the graft vessel, prevents the inner tissue of the graft vessel at the other side of the connector being damaged. In order to allow sufficient vessel length for closing the first end of the graft vessel after establishing the anastomosis connection, the distance from the connector to the first end is at least 0.5 cm. When a catheter - like an Excimer laser catheter as is for example disclosed in EP-750476 and US 5964750 - is used for making the fluid connection between the lumens of the graft vessel and recipient vessel, the distance from the connector to the first end is at least 1.0 cm.

According to a further embodiment of the assembly according to the invention, a rod like application member could be inserted through an end of the graft vessel into the lumen of the graft vessel.

According to the second aspect, the invention provides a method of making an assembly comprising on the one hand a graft vessel and on the other hand an anastomosis connector; wherein the graft is during the complete method of making the assembly, separated from human or animal body; wherein the connector comprises a ring and two pins; each pin having a free end and a fixed end connected to the ring; the pins extending, viewed from the fixed end towards the free end, next to and set apart from each other; each pin having an overlap section where the pin, viewed in axial direction of the ring, overlaps the plane bounded by the outer contour of the ring;
wherein the method comprises the step of:
- arranging the ring to lie, with its full circumference against the wall of the graft vessel and with its axial axis transverse to the wall of the graft vessel; and
- attaching the connector to the graft vessel.

With this method an assembly according to the invention can be made.

According to a further embodiment of the method of the second aspect of the invention, further comprising the step of making an opening through the wall of the graft vessel in the region surrounded by the ring. As already indicated above, the step of making said opening can comprise burning with a laser, such as an Excimer laser, an opening through the wall of the graft vessel.

According to a further embodiment of the method of the second aspect of the invention, the step of attaching the connector to the graft vessel comprises:
- suturing the ring onto the wall of the graft vessel; and/or
- adhering the ring onto the wall of the graft vessel; and/or
- piercing the pins through the wall of the graft vessel.

According to a further embodiment of the method of the second aspect of the invention, the step of attaching the connector to the graft vessel comprises:
- inserting the connector into the lumen of the graft vessel with the free ends of the pins pointing in the direction of insertion;
- piercing the free ends of the pins from the inside of the graft vessel through the wall of the graft vessel to outside the graft vessel and subsequently shifting the connector further in the direction of insertion up to the pins fully passed through the wall of the graft vessel and the ring lies against the inner surface of the wall of the graft vessel.

According to a further embodiment of the method of the second aspect of the invention the ring is placed against the outside of the graft vessel when performing the step of attaching the connector to the graft vessel. In this embodiment, the step of attaching the connector to the graft vessel can comprise the step of covering the inside of the ring with a part of the wall of the graft vessel lying inside the region surrounded by the ring.

The invention will now be further elucidated with reference to the drawings. In these drawings:
- Figure 1 shows a schematic view of a human heart with two bypasses;
- Figure 2 shows a schematic perspective view of two connector as can be used with the invention; figure 2a shows one example and figure 2b shows another example;
- Figure 3 illustrates with schematic perspective views a first method of making an assembly according to the invention, the figures 3a, 3b, 3c and 3d each illustrate subsequent steps;
- Figure 4 illustrates with schematic perspective views a second method of making an assembly according to the invention, the figures 4a and 4b each illustrate subsequent steps; and
- Figure 5 illustrates with schematic perspective views of an exemplary method of making a side to side anastomosis, the figures 5a, 5b, 5c and 5b each illustrate subsequent steps.

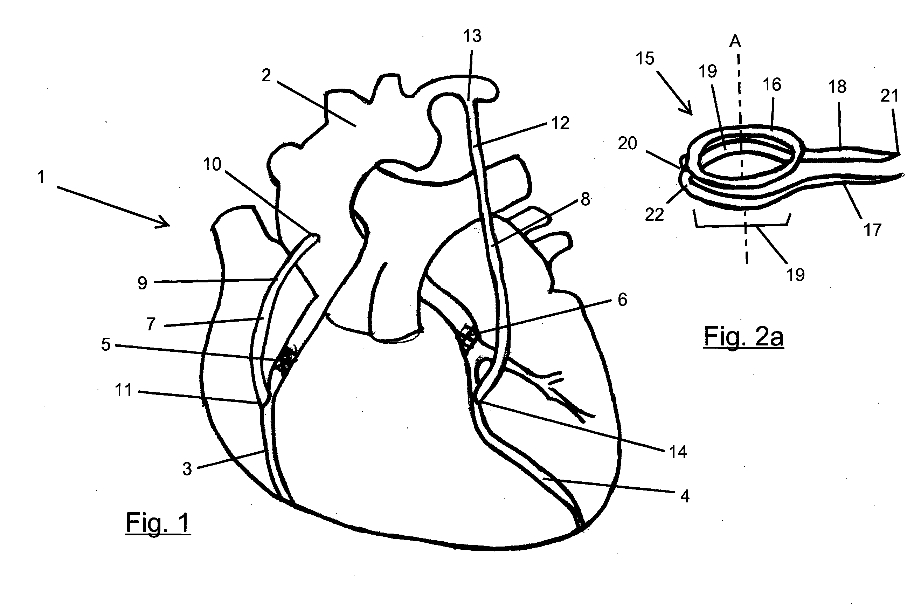

Figure 1 shows schematically a human heart 1. No. 2 indicates the aorta and no. 3 and no. 4 are both a coronary artery which is blocked at 5 respectively 6. In order to restore circulation through the blocked coronary arteries 3 and 4, a bypass 7 has been made to bypass blockage 5 and a bypass 8 has been made to bypass blockage 6.

For bypass 7 a saphenous vein from the leg has been used as graft vessel 9. The anastomotic connections at 10 and 11 are made and will be described further below. Graft vessel 9 is prepared according to the invention completely outside and separated from the body of the patient. This preparation can for example take place at a location where the patient is not present, but it can also take place close to the patient in the operating room.

The term 'the graft vessel being separated from a human or animal body' and the term 'the graft vessel being separated from the body of the patient' as used in this application means that the graft vessel is a separate entity not attached to a human/animal/patient body. Of coarse these term(s) do not exclude that somebody can keep it in his hand or manipulate it. These term(s) mean that the graft is, when separated, not part of the biological system of a body of a human/animal/patient.

For bypass 8 an internal mammary artery from the chest has been used as graft vessel 12. At location 13 this graft vessel has a natural connection to the aorta. Thus at this location no artificial anastomotic connection is required. At location 14 an exemplary anastomotic connection has been made. Graft vessel 12 may be prepared inside the body of the patient or completely outside the body of the patient, but graft vessel 12 will stay connected at location 13 with the patient.

In figure 1 the coronary artery 4 is the recipient vessel with respect to graft vessel 12 and coronary artery 3 and aorta 2 are each a recipient vessels with respect to graft vessel 9.

Figure 2a shows schematically a connector 15 that can be used according to the invention. This connector 15 comprises a ring 16 and two pins 17, 18 which are with the so called fixed end 22 attached to the ring 16 at 20, and extend from the fixed end 20 towards the pointed free end 21, next to and set apart from each other in the same direction. The pins 17, 18 have an overlap section 19 in which they overlap the plane bounded by the ring. The plane bounded by the ring is as such defined as the plane 'surface' lying inside the ring and the ring itself. The plane bounded by the ring thus does not extend outside the ring. Axis A is the axial axis of the ring.

A connector 15 like the one as shown in figure 2a is also known from WO 2009/123434. All the securing devices as disclosed in WO 2009/12434 can be used as a connector for the present invention.

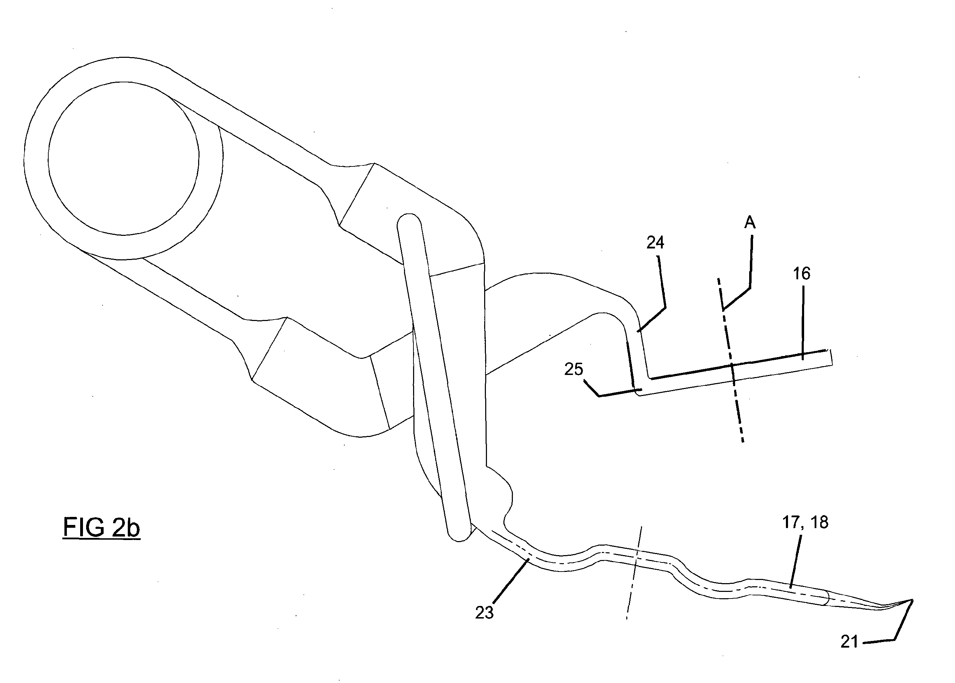

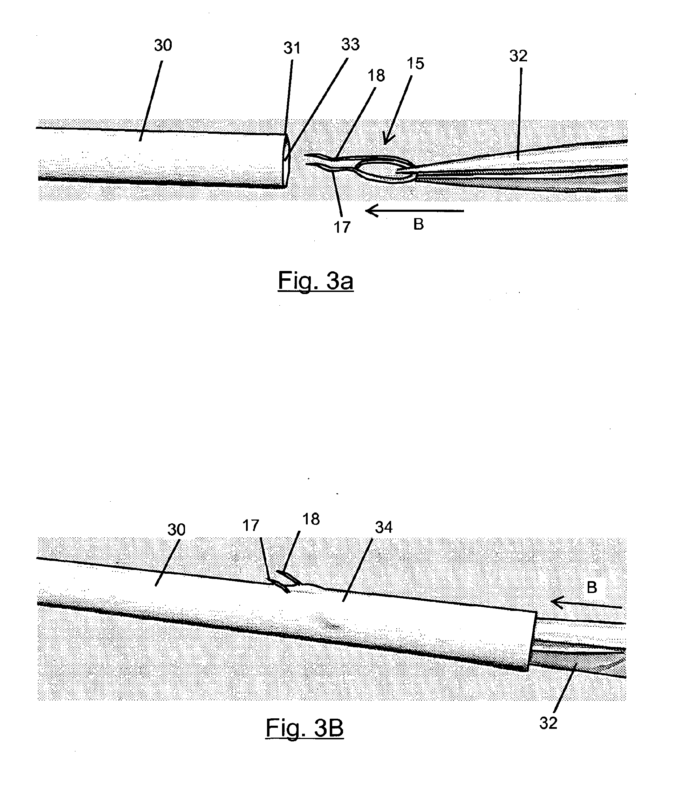

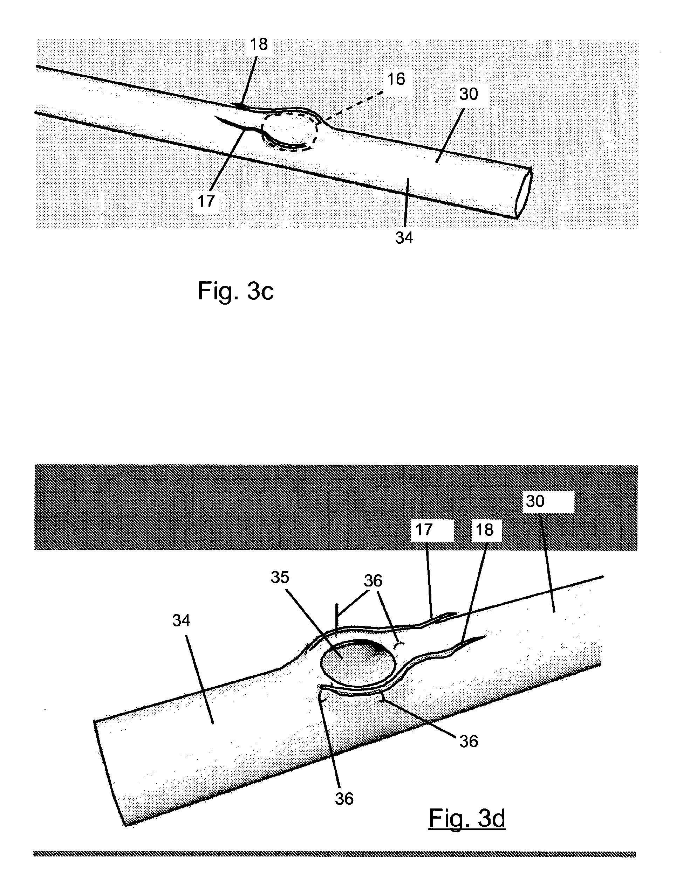

Although the connector 15 shown in figure 2a is also shown in the figures 3, 4 and 5 - to be discussed below, it is noted that connectors of the according to the invention can also be of different design, as follows from the example of the connector shown in figure 2b. For details of figure 2b reference is made to WO 2011/062495.

Referring to figure 2b of the present application, it might be considered to extend 23 and to arrange at 25 between the ring 16 and leg 24 a similar extension about parallel to 23 in order to create more distance between the ring 16 and pins 17, 18 and the leg 24.

Figure 3 illustrates a first method of making an assembly according to the invention.

Figure 3a shows a graft vessel 30, the connector 15 of figure 2a and the gripper end of a forceps 32. The forceps 32 holds the connector 15 and inserts the connector 15 in the direction of arrow B into the lumen 33 of graft vessel 30.

Figure 3b shows that the pins 17 and 18 are pierced from inside the lumen of graft vessel 30 through the wall 34 of the graft vessel 30 to outside the graft vessel 30. The insertion movement in the direction B is continued to shift the connector further in the direction of insertion B up to the pins fully passed through the wall 34 of the graft vessel, see figure 3c.

In figure 3c, the pins have fully passed through the wall 34 of the graft vessel 30 and lie essentially parallel to the wall of the graft vessel 30. The ring 16 of the connector lies inside the graft vessel and is shown by dashed line for illustrative purpose.

Figure 3d shows that, in the region surrounded by the ring 16, an opening 35 has been made through the wall 34 of the graft vessel 30. This opening serves to allow blood to enter or leave the graft vessel at this place in order to allow blood flowing into or from the recipient vessel. This opening 35 can be made with an Excimer laser as mentioned earlier several times. The opening can also be made with another tool like a knife.

Figure 3d further shows that sutures 36 can be used to attach the ring 16 against the inner side of the wall 34 of the graft vessel 30. The sutures 36 can be provided before or after making the opening 35.

It is to be noted that the opening 35 does not need to be made at this stage, it is also conceivable to make the opening after attaching the assembly of graft vessel 30 and connector 15 to the recipient vessel.

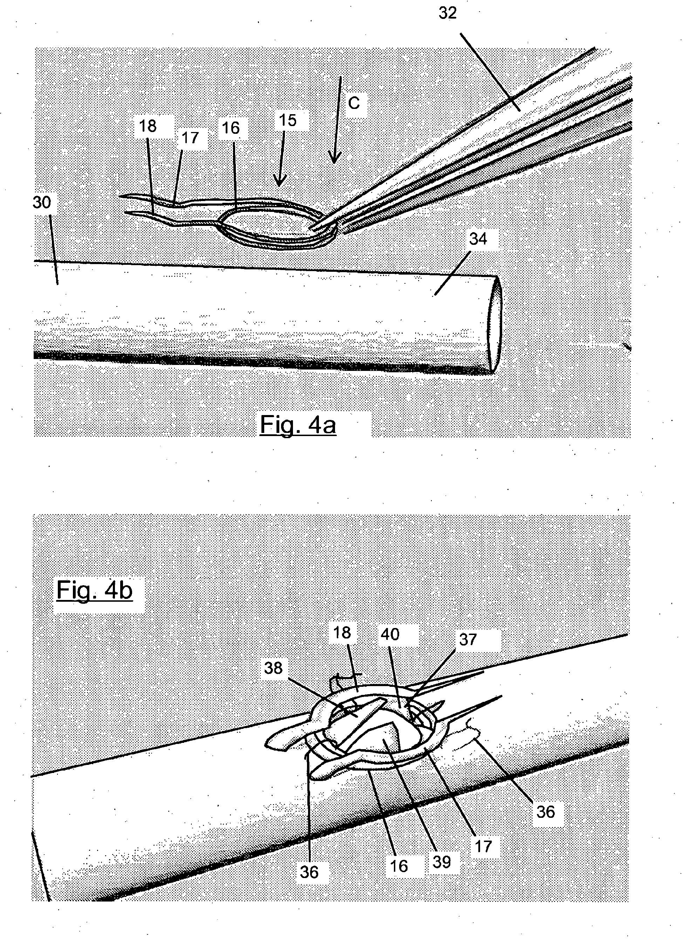

Figure 4 illustrates a second method of making an assembly according to the invention. The main difference between the method illustrated with figure 3 and the method illustrated with figure 4, is that in the method of figure 3 the ring of the connector is arranged inside the graft vessel and the pins 17, 18 of the connector are arranged outside the graft vessel, whilst in the method of figure 4 the ring and pins are both arranged outside the graft vessel.

Figure 4a shows a graft vessel 30, the connector 15 of figure 2a and the gripper end of a forceps 32. The forceps 32 holds the connector 15 and places the connector 15 in the direction of arrow C against the graft vessel 30 so that the ring 16 lies between the wall of the graft vessel 30 and the pins 17, 18.

Fig 4b shows as a next step that an opening 35 has been made in the region of the wall of the graft vessel which surrounded by the ring. This opening 35 will later allow blood flow between the graft vessel and recipient vessel. Further the wall tissue of the graft vessel 30 in said region surrounded by the ring 16 is not removed, at least not fully removed. Parts 37, 38, and 39 of the wall tissue in said region are used to cover the inner side of the ring 16. Doing so the internal surface 40 of the parts 37, 38, 39 of wall tissue will face inwardly. After completing the anastomotic connection, it will be this internal surface 40 which contacts blood flowing through the anastomotic connection. This is favourable as this internal surface is the natural surface for contacting blood. As shown sutures 36 are used on the one hand to attach the ring 16 to the graft vessel and on the other hand to keep the parts 37, 38 and 39 against the inner side of the ring. It is noted that in case the parts 37, 38, 39 have, in radial direction of the ring, sufficient length, they might be folded around the ring and inserted into the gap between the ring 16 and pins 17, 18.

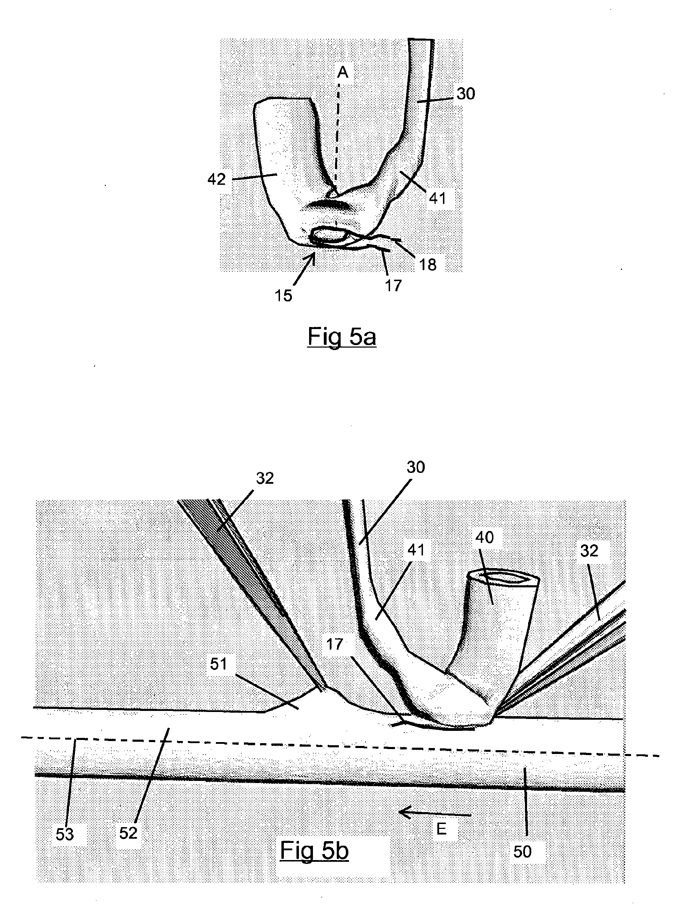

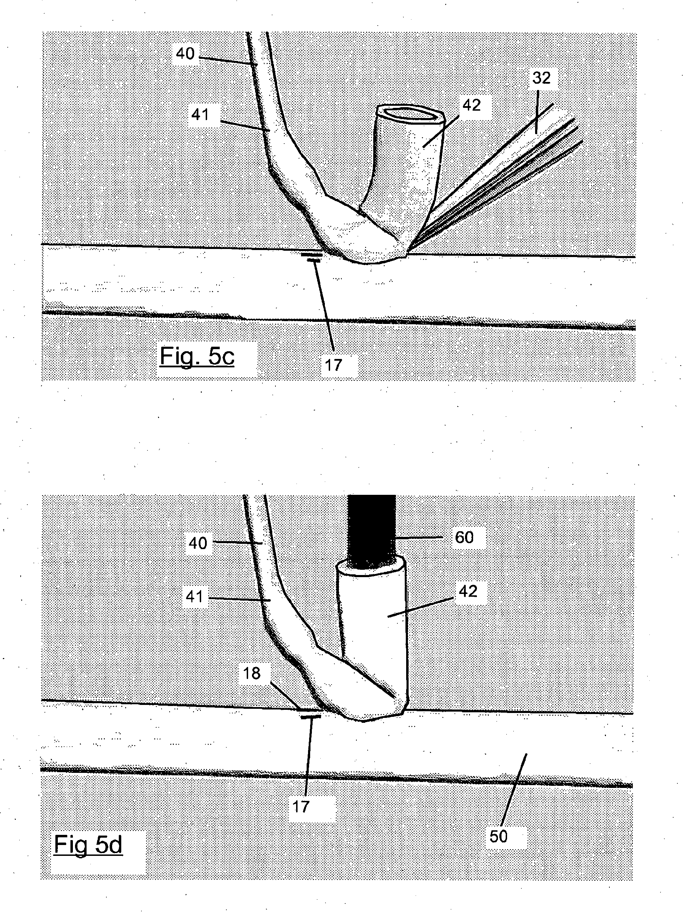

Figure 5 shows how to use an assembly according to the invention for attachment to a recipient vessel in order to accomplish a side-to-side anastomosis. Although the assembly shown in figure 5 is an assembly made according to figure 4, it is noted that an assembly made according to figure 3 can also be attached to the recipient vessel in the manner as illustrated in figure 5.

In figure 5a, the parts 41 and 42 of the graft vessel 30, which lie adjacent the connector 15, may be folded towards the axial axis A of the ring 16, or said differently these parts 41 and 42 may be folded towards each other. This may provide the operator, who has to make the exemplary anastomotic connection, a better view onto the connector 15.

Figure 5b shows in addition a recipient vessel 50 and two forceps 32. One of the forceps holds the connector 15 at its ring. The other foreceps grips a part 51 of the vessel wall 52 of the recipient vessel and pulls this part 51 away from the longitudinal axis 53 of the recipient vessel 50. Although this gripping and pulling of the part 51 is optional, it makes it easier to pierce the pins 17 and 18 into the wall of the recipient vessel.

In order to pierce the pins 17 and 18 from outside the recipient vessel 50 through the wall 52 of the recipient vessel into the lumen of the recipient vessel, the assembly of graft vessel 30 and connector 15 may be shifted in the direction of arrow D. Once the pointed free ends of the pins passed from the outside through the vessel wall 52, the connector may be shifted further in the direction of arrow D. At the end of this shifting movement, the free ends of the pins 17, 18 can be pierced again through the vessel wall, but now from the inside to the outside. This condition is shown in figure 5d.

Figure 5d further shows that a laser catheter 60 may be inserted into the part 42 of the graft vessel 40 to burn away that part of the wall of the recipient vessel lying in the region surrounded by the ring 16. For details of a suitable laser catheter 60 reference can be made to the earlier mentioned US 5,964,750. US 5,964,750 show in more detail the manner of function of the laser catheter tip during application of laser light. A ring shaped cut is burnt and the vessel part lying inside this cut may be suctioned against a grid to remove it. After the opening for connection of the lumens of the graft vessel and recipient vessel have been made, the laser catheter may be withdrawn in direction E and the part 42 may be provided with a clip to prevent leakage of blood. Part 42 can also be closed by suturing, burning or other means in order to prevent blood from flowing out this end. During use of the laser catheter 60 also the part 41 of the graft vessel might be temporarily closed, for example by a clip.

It is noted that in an exemplary case where the graft vessel has sufficient length, it may be also possible to use one graft vessel for a double bypass, also called a jump. Referring to figure 1, it may be for example conceivable to connect one end of the graft vessel at 10 to the aorta, the other end at 14 to the coronary artery 4 and to connect the graft vessel somewhere in between its ends at the location 11 with coronary artery 3.

## Claims

1. Assembly comprising a graft vessel (30) and an anastomosis connector (15);
wherein the graft vessel (30) is separated from a human or animal body;
wherein the connector (15) comprises a ring (16) and two pins (17, 18);
each pin (17, 18) having a free end (21) and a fixed end (22) connected to the ring (16);
the pins (17, 18) extending, viewed from the fixed end (22) towards the free end (21), next to and set apart from each other;
each pin (17, 18) having an overlap section (19) where the pin, viewed in axial direction of the ring (16), overlaps the plane bounded by the outer contour of the ring (16);
wherein the connector (15) is attached to the graft vessel (30);
wherein the pins (17, 18) are arranged outside the graft vessel (30); and
wherein the ring (16) lies, with its full circumference, against the wall (34) of the graft vessel (30);
**characterized, in that** the axial axis of the ring (16) extends transverse to the wall (34) of the graft vessel (30).

2. Assembly according to claim 1, wherein the wall (34) of the graft vessel (30) is provided with an opening (35) surrounded by the ring (16), and wherein the opening (35) preferably has been formed by laser cutting an opening (35) into the wall (34) of the graft vessel (30).

3. Assembly according to one of the preceding claims, wherein the connector (15) is attached to the graft vessel (30) by sutures (36) attaching the ring (16) onto the wall (34) of the graft vessel (30), and/or by adhesive adhering (16) the ring (16) onto the wall (34) of the graft vessel (30), and/or by the pins (17, 18) piercing through the wall (34) of the graft vessel (30).

4. Assembly according to one of the preceding claims, wherein the pins (17, 18) extend in the longitudinal direction of the graft vessel (30).

5. Assembly according to one of the preceding claims, wherein the ring (16) lies against the outer surface of the wall (34) of the graft vessel (30), and wherein a part of the wall (34) of the graft vessel (30) lying inside the region surrounded by the ring (16) is folded outwardly to cover the inner side of the ring (16).

6. Assembly according to one of the preceding claim 1-4, wherein the ring (16) lies against the inner surface of the wall (34) of the graft vessel (30) and wherein the pins (17, 18) lie outside the graft vessel (30) and extend parallel to the outer surface of the wall (34) of the graft vessel (30).

7. Assembly according to one of the preceding claims, wherein the graft vessel (30) has a first end and a second end; wherein the pins (17, 18), viewed from the fixed ends (22) towards the free ends (21), extend towards the second end of the graft vessel (30), and wherein the distance from the connector (15) to the first end of the graft vessel (30) is smaller than the distance from the connector (15) to the second end, and wherein this distance from the connector (15) to the first end is preferably at least 0.5 cm, such 1.0 cm or more.

8. Assembly according to one of the preceding claims, wherein, in a first position of the pins (17, 18) with respect to the ring (16), at least part of the overlap sections (19) extend parallel to the ring (16).

9. Assembly according to one of the preceding claims, wherein the pins (17, 18) are rigidly connected to the ring (16).

10. Assembly according to one of the claims 1-8, wherein the pins (17, 18) are with respect to the ring (16) moveable from a first position to a second position in order to form between the ring (16) and the pins (17, 18) an opened jaw; and in which the pins (17, 18) are moveable from the second position to the first position towards the ring (16) in order to close the jaw; and
wherein preferably
in the second position the overlap sections (19) of the pins (17, 18) are angled with respect to the plane defined by the ring (16) in order to form said jaw which, viewed from the fixed end (22) towards the free end (21), widens, and wherein the pins (17, 18) are swivelable, with respect to the ring (16), from the second position to the first position;
and/or
wherein the connector (15) further comprises a tensioning member which, when the pins (17, 18) are in the second position, pretensions the pins (17, 18) towards the first position.

11. Assembly according to one of the preceding claims, wherein the free ends (21) of the pins (17, 18) are arranged at a distance from the overlap section (19), wherein said distance is at least 50% of the radius of the ring (16), preferably at least 100% of the radius of the ring (16); and wherein the sections of the pins (17, 18) between the overlap section (19) and the free end (21) of each pin, are preferably substantially parallel to each other.

12. Method of making an assembly comprising a graft vessel (30) and an anastomosis connector (15);
wherein the graft is, during the complete method, of making the essembly separated from human or animal body;
wherein the connector (15) comprises a ring (16) and two pins (17, 18); each pin (17, 18) having a free end (21) and a fixed end (22) connected to the ring (16); the pins (17, 18) extending, viewed from the fixed end (22) towards the free end (21), next to and set apart from each other; each pin (17, 18) having an overlap section (19) where the pin, viewed in axial direction of the ring (16), overlaps the plane bounded by the outer contour of the ring (16);
wherein the method comprises the step of:
• arranging the ring (16) to lie, with its full circumference against the wall (34) of the graft vessel (30) and with its axial axis transverse to the wall (34) of the graft vessel (30); and
• attaching the connector (15) to the graft vessel (30).

13. Method according to claim 12, further comprising the step of:
• making an opening (35) through the wall (34) of the graft vessel (30) in the region surrounded by the ring (16);
wherein the step of making said opening (35) is preferably made by burning with a laser, such as an Excimer laser, an opening (35) through the wall (34) of the graft vessel (30).

14. Method according to one of claims 13-15, wherein the step of attaching the connector (15) to the graft vessel (30) comprises suturing (16) the ring (16) onto the wall (34) of the graft vessel (30); and/or adhering (16) the ring (16) onto the wall (34) of the graft vessel (30); and/or piercing the pins (17, 18) through the wall (34) of the graft vessel (30).

15. Method according to one of claims 12-14, wherein the step of attaching the connector (15) to the graft vessel (30) comprises:
• inserting the connector (15) into the lumen of the graft vessel (30) with the free ends (21) of the pins (17, 18) pointing in the direction of insertion;
• piercing the free ends (21) of the pins (17, 18) from the inside of the graft vessel (30) through the wall (34) of the graft vessel (30) to outside the graft vessel (30) and subsequently shifting the connector (15) further in the direction of insertion up to the pins (17, 18) fully passed through the wall (34) of the graft vessel (30) and the ring (16) lies against the inner surface of the wall (34) of the graft vessel (30).

16. Method according to one of claims 12-14, wherein, in the step of attaching the connector (15) to the graft vessel (30), the ring (16) is placed against the outside of the graft vessel (30); and the step of attaching the connector (15) to the graft vessel (30) further comprises covering (16) the inside of the ring (16) with a part of the wall (34) of the graft vessel (30) lying inside the region surrounded by the ring (16).

## Patentansprüche

1. Anordnung umfassend ein Transplantatgefäß (30) und einen Anastomoseverbinder (15);
wobei das Transplantatgefäß (30) von einem menschlichen oder tierischen Körper getrennt ist;
wobei der Verbinder (15) einen Ring (16) und zwei Stifte (17, 18) umfasst;
wobei jeder Stift (17, 18) ein freies Ende (21) und ein befestigtes Ende (22), welches mit dem Ring (16) verbunden ist, aufweist;
wobei sich die Stifte (17, 18) aus Sicht des befestigten Endes (22) in Richtung des freien Endes (21) nebeneinander und voneinander beabstandet erstrecken;
wobei jeder Stift (17, 18) einen Überlappungsabschnitt (19) aufweist, wo der Stift in axialer Richtung des Rings (16) betrachtet die Ebene überlappt, welche durch die äußere Kontur des Rings (16) begrenzt wird;
wobei der Verbinder (15) an dem Transplantatgefäß (30) angebracht ist;
wobei die Stifte (17, 18) außerhalb des Transplantatgefäßes (30) angeordnet sind; und
wobei der Ring (16) mit seinem vollen Umfang an der Wand (34) des Transplantatgefäßes (30) liegt;
**dadurch gekennzeichnet, dass** sich die axiale Achse des Rings (16) quer zu der Wand (34) des Transplantatgefäßes (30) erstreckt.

2. Anordnung nach Anspruch 1, wobei die Wand (34) des Transplantatgefäßes (30) mit einer Öffnung (35) versehen ist, welche den Ring (16) umgibt, und wobei die Öffnung (35) vorzugsweise mit einem Laser ausgebildet wurde, welcher eine Öffnung (35) in die Wand (34) des Transplantatgefäßes (30) schneidet.

3. Anordnung nach einem der vorherigen Ansprüche, wobei der Verbinder (15) mittels chirurgischer Nähte (36), welche den Ring (16) auf der Wand (34) des Transplantatgefäßes (30) anbringen, und/oder mittels Klebstoff, welcher den Ring (16) auf der Wand (34) des Transplantatgewebes (30) klebt, und/oder mittels der Stifte (17, 18), welche durch die Wand (34) des Transplantatgefäßes (30) dringen, an dem Transplantatgefäß (30) angebracht ist.

4. Anordnung nach einem der vorhergehenden Ansprüche, wobei sich die Stifte (17, 18) in der Längsrichtung des Transplantatgefäßes (30) erstrecken.

5. Anordnung nach einem der vorhergehenden Ansprüche, wobei der Ring (16) an der Außenfläche der Wand (34) des Transplantatgefäßes (30) liegt, und wobei ein Teil der Wand (34) des Transplantatgefäßes (30), welcher innerhalb des von dem Ring (16) umgebenen Bereichs liegt, nach außen gefaltet ist, um die Innenseite des Rings (16) zu bedecken.

6. Anorndung nach einem der vorhergehenden Ansprüche 1 bis 4, wobei der Ring (16) an der inneren Fläche der Wand (34) des Transplantatgefäßes (30) liegt und wobei die Stifte (17, 18) außerhalb des Transplantatgefäßes (30) liegen und sich parallel zu der Außenfläche der Wand (34) des Transplantatgefäßes (30) erstrecken.

7. Anordnung nach einem der vorhergehenden Ansprüche, wobei das Transplantatgefäß (30) ein erstes Ende und ein zweites Ende aufweist; wobei sich die Stifte (17, 18) von den festen Enden (22) in Richtung der freien Enden (21) betrachtet in Richtung des zweiten Endes des Transplantatgefäßes (30) erstrecken und wobei der Abstand von dem Verbinder (15) zu dem ersten Ende des Tramsplantatgefäßes (30) kleiner als der Abstand von dem Verbinder (15) zu dem zweiten Ende ist, und wobei dieser Abstand von dem Verbinder (15) zu dem ersten Ende vorzugsweise mindestens 0,5 cm, wie z. B. 1,0 cm oder mehr, beträgt.

8. Anordnung nach einem der vorhergehenden Ansprüche, wobei sich in einer ersten Position der Stifte (17, 18) bezogen auf den Ring (16) zumindest ein Teil des Überlappungsbereichs (19) parallel zu dem Ring (16) erstreckt.

9. Anordnung nach einem der vorhergehenden Ansprüche, wobei die Stifte (17, 18) fest mit dem Ring (16) verbunden sind.

10. Anordnung nach einem der Ansprüche 1 bis 8, wobei die Stifte (17, 18) bezogen auf den Ring (16) von einer ersten Position zu einer zweiten Position beweglich sind, um zwischen dem Ring (16) und den Stiften (17, 18) eine geöffnete Klaue auszubilden; und wobei die Stifte (17, 18) von der zweiten Position zu der ersten Position in Richtung des Rings (16) beweglich sind, um die Klaue zu schliessen; und
wobei vorzugsweise
in der zweiten Position die Überlappungsabschnitte (19) der Stifte (17, 18) bezogen auf die durch den Ring (16) definierte Ebene abgewinkelt sind, um die Klaue auszubilden, welche von dem festen Ende (22) in Richtung des freien Endes (21) betrachtet breiter wird, und wobei die Stifte (17, 18) bezogen auf den Ring (16) von der zweiten Position zu der ersten Position drehbar sind;
und/oder
wobei der Verbinder (15) ferner ein Spannelement umfasst, welches die Stifte (17, 18) in Richtung der ersten Position vorspannt, wenn die Stifte (17, 18) in der zweiten Position sind.

11. Anordnung nach einem der vorhergehenden Ansprüche, wobei die freien Enden (21) der Stifte (17,18) in einem Abstand von dem Überlappungsabschnitt (19) angeordnet sind, wobei der Abstand mindestens 50% des Radius des Rings (16), vorzugsweise mindestens 100% des Radius des Rings (16), beträgt; und wobei die Abschnitte der Stifte (17, 18) zwischen dem Überlappungsabschnitt (19) und dem freien Ende (21) eines jeweiligen Stifts vorzugsweise im wesentlichen parallel zueinander sind.

12. Verfahren zum Herstellen einer Anordnung, welche ein Transplantatgefäß (30) und einen Anastomoseverbinder (15) umfasst;
wobei das Transplantat während des gesamten Verfahrens des Herstellens der Anordnung von einem menschlichen oder tierischen Körper getrennt ist;
wobei der Verbinder (15) einen Ring (16) und zwei Stifte (17, 18) umfasst; wobei jeder Stift (17, 18) ein freies Ende (21) und ein festes Ende (22), welches mit dem Ring (16) verbunden ist, aufweist; wobei sich die Stifte (17, 18) von dem festen Ende (22) in Richtung des freien Endes (21) betrachtet nebeneinander und voneinander beabstandet erstrecken; wobei jeder Stift (17, 18) einen Überlappungsabschnitt (19) aufweist, wo der Stift in axialer Richtung des Rings (16) betrachtet die durch die äußere Kontur des Rings (16) begrenzte Ebene überlappt;
wobei das Verfahren die Schritte umfasst:
• Anordnen des Rings (16), um mit seinem vollen Umfang an der Wand (34) des Transplantatgefäßes (30) und mit seiner axialen Achse quer zu den Wand (34) des Transplantatgefäßes (30) zu liegen; und
• Anbringen des Verbinders (16) an dem Transplantatgefäß (30).

13. Verfahren nach Anspruch 12, ferner umfassend den Schritt:
• Anfertigen einer Öffnung (35) durch die Wand (34) des Transplantatgefäßes (30) in dem durch den Ring (16) umgebenen Bereich;
wobei der Schritt des Anfertigens der Öffnung (35) vorzugsweise ausgeführt wird, indem mit einem Laser, wie z.B. einem Excimer-Laser, eine Öffnung (35) durch die Wand (34) des Transplantatgefäßes (30) gebrannt wird.

14. Verfahren nach einem der Ansprüche 12 bis 13, wobei der Schritt des Anbringens des Verbinders (15) an dem Transplantatgefäß (30) ein Nähen (16) des Rings (16) auf die Wand (34) des Transplantatgefäßes (30); und/oder ein Kleben (16) des Rings (16) auf die Wand (34) des Transplantatgefäßes (30); und/oder ein Durch-dringen der Stifte (17, 18) durch die Wand (34) des Transplantatgefäßes (30) umfasst.

15. Verfahren nach einem der Ansprüche 12 bis 14, wobei der Schritt des Anbringens des Verbinders (15) an dem Transplantatgefäß (30) umfasst:
• Einsetzen des Verbinders (15) in das Lumen des Transplantatgefäßes (30), wobei die freien Enden (21) der Stifte (17, 18) in die Richtung der Einsetzung zeigen;
• Durchdringen der freien Enden (21) der Stifte (17, 18) von der Innenseite des Transplantatgefäßes (30) durch die Wand (34) des Transplantatgefäßes (30) zu einer Außenseite des Transplantatgefäßes (30) und nachfolgendes Schieben des Verbinders (15) weiter in die Richtung der Einsetzung bis die Stifte (17, 18) vollständig durch die Wand (34) des Transplantatgefäßes (30) durchgeführt sind und der Ring (16) an der Innenfläche der Wand (34) des Transplantatgefäßes (30) liegt.

16. Verfahren nach einem der Ansprüche 12 bis 14, wobei bei dem Schritt des Anbringens des Verbinders (15) an dem Transplantatgefäß (30) der Ring (16) an der Außenseite des Transplantatgefäßes (30) angeordnet wird; und wobei der Schritt des Anbringens des Verbinders (15) an dem Transplantatgefäß (30) ferner ein Bedecken (16) der Innenseite des Rings (16) mit einem Teil der Wand (34) des Transplantatgefäßes (30), welcher innerhalb des von dem Ring (16) umgebenen Bereichs liegt, umfasst.

## Revendications

1. Ensemble comprenant un vaisseau de greffe (30) et un connecteur d'anastomose (15) ;
dans lequel le vaisseau de greffe (30) est séparé d'un corps humain ou de celui d'un animal ;
dans lequel le connecteur (15) comprend un anneau (16) et deux broches (17, 18) ;
chaque broche (17, 18) comportant une extrémité libre (21) et une extrémité fixée (22) reliée à l'anneau (16) ;
les broches (17, 18) s'étendant, lorsqu'elles sont vues de l'extrémité fixée (22) vers l'extrémité libre (21), l'une à côté de l'autre et séparées l'une de l'autre ;
chaque broche (17, 18) comportant une section de recouvrement (19) où la broche, lorsqu'elle est vue dans une direction axiale de l'anneau (16), recouvre le plan délimité par le contour extérieur de l'anneau (16) ;
dans lequel le connecteur (15) est attaché au vaisseau de greffe (30) ;
dans lequel les broches (17, 18) sont agencées à l'extérieur du vaisseau de greffe (30) ; et
dans lequel l'anneau (16) se trouve, avec sa circonférence entière, contre la paroi (34) du vaisseau de greffe (30) ;
**caractérisé en ce que** l'axe axial de l'anneau (16) s'étend transversalement à la paroi (34) du vaisseau de greffe (30).

2. Ensemble selon la revendication 1, dans lequel la paroi (34) du vaisseau de greffe (30) est pourvue d'une ouverture (35) entourée par l'anneau (16), et dans lequel l'ouverture (35) a été de préférence formée par découpe au laser d'une ouverture (35) dans la paroi (34) du vaisseau de greffe (30).

3. Ensemble selon l'une des revendications précédentes, dans lequel le connecteur (15) est attaché au vaisseau de greffe (30) par des points de suture (36) attachant l'anneau (16) sur la paroi (34) du vaisseau de greffe (30), et/ou en faisant adhérer par adhésif l'anneau (16) sur la paroi (34) du vaisseau de greffe (30), et/ou par les broches (17, 18) transperçant la paroi (34) du vaisseau de greffe (30).

4. Ensemble selon l'une des revendications précédentes, dans lequel les broches (17, 18) s'étendent dans la direction longitudinale du vaisseau de greffe (30).

5. Ensemble selon l'une des revendications précédentes, dans lequel l'anneau (16) se trouve contre la surface extérieure de la paroi (34) du vaisseau de greffe (30), et dans lequel une partie de la paroi (34) du vaisseau de greffe (30) se trouvant à l'intérieur d'une région entourée par l'anneau (16) est repliée vers l'extérieur pour recouvrir le côté intérieur de l'anneau (16).

6. Ensemble selon l'une des revendications 1 à 4 précédentes, dans lequel l'anneau (16) se trouve contre la surface intérieure de la paroi (34) du vaisseau de greffe (30), et dans lequel les broches (17, 18) se trouvent à l'extérieur du vaisseau de greffe (30) et s'étendent parallèlement à la surface extérieure de la paroi (34) du vaisseau de greffe (30).

7. Ensemble selon l'une des revendications précédentes, dans lequel le vaisseau de greffe (30) comporte une première extrémité et une deuxième extrémité ; dans lequel les broches (17, 18), lorsqu'elles sont vues des extrémités fixées (22) vers les extrémités libres (21), s'étendent vers la deuxième extrémité du vaisseau de greffe (30), et dans lequel la distance du connecteur (15) à la première extrémité du vaisseau de greffe (30) est inférieure à la distance du connecteur (15) à la deuxième extrémité, et dans lequel cette distance du connecteur (15) à la première extrémité est de préférence d'au moins 0,5 cm, telle que de 1,0 cm ou plus.

8. Ensemble selon l'une des revendications précédentes, dans lequel, dans une première position des broches (17, 18) par rapport à l'anneau (16), au moins une partie des sections de recouvrement (19) s'étend parallèlement à l'anneau (16).

9. Ensemble selon l'une des revendications précédentes, dans lequel les broches (17, 18) sont reliées rigidement à l'anneau (16).

10. Ensemble selon l'une des revendications 1 à 8, dans lequel les broches (17, 18) peuvent être déplacées, par rapport à l'anneau (16), d'une première position à une deuxième position afin de former, entre l'anneau (16) et les broches (17, 18), une mâchoire ouverte ; et dans lequel les broches (17, 18) peuvent être déplacées de la deuxième position à la première position vers l'anneau (16) afin de fermer la mâchoire ; et
dans lequel, de préférence,
dans la deuxième position, les sections de recouvrement (19) des broches (17, 18) sont inclinées par rapport au plan défini par l'anneau (16) afin de former ladite mâchoire qui, lorsqu'elle est vue de l'extrémité fixée (22) vers l'extrémité libre (21), s'élargit, et dans lequel les broches (17, 18) peuvent pivoter, par rapport à l'anneau (16), de la deuxième position à la première position ;
et/ou
dans lequel le connecteur (15) comprend en outre un élément de tension qui, lorsque les broches (17, 18) sont dans la deuxième position, applique une pré-tension aux broches (17, 18) vers la première position.

11. Ensemble selon l'une des revendications précédentes, dans lequel les extrémités libres (21) des broches (17, 18) sont agencées à une distance de la section de recouvrement (19), dans lequel ladite distance est égale à au moins 50 % du rayon de l'anneau (16), de préférence à au moins 100 % du rayon de l'anneau (16) ; et dans lequel les sections des broches (17, 18) entre la section de recouvrement (19) et l'extrémité libre (21) de chaque broche sont de préférence sensiblement parallèles l'une à l'autre.

12. Procédé de fabrication d'un ensemble comprenant un vaisseau de greffe (30) et un connecteur d'anastomose (15) ;
dans lequel la greffe est, pendant le procédé complet de fabrication de l'ensemble, séparée d'un corps humain ou de celui d'un animal ;
dans lequel le connecteur (15) comprend un anneau (16) et deux broches (17, 18) ; chaque broche (17, 18) ayant une extrémité libre (21) et une extrémité fixée (22) reliée à l'anneau (16) ; les broches (17, 18) s'étendant, lorsqu'elles sont vues de l'extrémité fixée (22) vers l'extrémité libre (21), l'une à côté de l'autre et séparées l'une de l'autre ; chaque broche (17, 18) comportant une section de recouvrement (19) où la broche, lorsqu'elle est vue dans une direction axiale de l'anneau (16), recouvre le plan délimité par le contour extérieur de l'anneau (16) ;
dans lequel le procédé comprend les étapes :
. d'agencement de l'anneau (16) pour qu'il soit avec sa circonférence entière contre la paroi (34) du vaisseau de greffe (30) et avec son axe axial transversal à la paroi (34) du vaisseau de greffe (30) ; et
. de fixation du connecteur (15) au vaisseau de greffe (30).

13. Procédé selon la revendication 12, comprenant en outre l'étape :
. de formation d'une ouverture (35) à travers la paroi (34) du vaisseau de greffe (30) dans la région entourée par l'anneau (16) ;
dans lequel l'étape de formation de ladite ouverture (35) est de préférence effectuée en brûlant avec un laser, tel qu'un laser à excimère, une ouverture (35) à travers la paroi (34) du vaisseau de greffe (30).

14. Procédé selon l'une des revendications 12 et 13, dans lequel l'étape de fixation du connecteur (15) au vaisseau de greffe (30) comprend la suture de l'anneau (16) sur la paroi (34) du vaisseau de greffe (30) ; et/ou l'adhérence de l'anneau (16) sur la paroi (34) du vaisseau de greffe (30) ; et/ou le transpercement de la paroi (34) du vaisseau de greffe (30) par les broches (17, 18).

15. Procédé selon l'une des revendications 12 à 14, dans lequel l'étape de fixation du connecteur (15) au vaisseau de greffe (30) comprend :
. l'insertion du connecteur (15) dans la lumière du vaisseau de greffe (30), les extrémités libres (21) des broches (17, 18) pointant dans la direction d'insertion ;
. le transpercement du vaisseau de greffe (30), par les extrémités libres (21) des broches (17, 18), de l'intérieur du vaisseau de greffe (30) à travers la paroi (34) du vaisseau de greffe (30) jusqu'à l'extérieur du vaisseau de greffe (30) et le décalage par la suite du connecteur (15) davantage dans la direction d'insertion jusqu'à ce que les broches (17, 18) aient entièrement traversé la paroi (34) du vaisseau de greffe (30) et que l'anneau (16) se trouve contre la surface intérieure de la paroi (34) du vaisseau de greffe (30).

16. Procédé selon l'une des revendications 12 à 14, dans lequel, à l'étape de fixation du connecteur (15) au vaisseau de greffe (30), l'anneau (16) est placé contre l'extérieur du vaisseau de greffe (30) ; et l'étape de fixation du connecteur (15) au vaisseau de greffe (30) comprend en outre le recouvrement de l'intérieur de l'anneau (16) par une partie de la paroi (34) du vaisseau de greffe (30) se trouvant à l'intérieur de la région entourée par l'anneau (16).
